# EUROPEAN PATENT APPLICATION

(11) **EP 4 129 391 A1**
(43) Date of publication of application: **08.02.2023**
(21) Application number: 21778725.8
(22) Date of filing: 16.02.2021
(51) Int. Cl.: A61M 37/00, A61K 41/00, A61N 2/02, A61N 2/00, A61F 7/12

(54) **NANOPARTICLE PROVISION MEDIUM FOR THERMOTHERAPY AND THERMOTHERAPY SYSTEM USING SAME**

(30) Priority: 03.04.2020 KR 20200040767
(71) Applicant: Seoul National University R&DB Foundation, Seoul 08826 (KR); Brain & Beyonds Co., Ltd., Seoul 03080 (KR)
(72) Inventor: PAEK, Sun Ha, Seoul 04422 (KR); PARK, Soon Beom, Seoul 04422 (KR)
(74) Representative: Algemeen Octrooi- en Merkenbureau B.V.
(86) International application number: PCT/KR2021/001954
(87) International publication number: WO 2021/201426

(57) **Abstract**

The technology disclosed in the present specification relates to a nanoparticle providing medium for hyperthermia, and the nanoparticle providing medium for hyperthermia according to an embodiment may be formed so as to capture nanoparticles in a region or one or more divided regions and block contact between the nanoparticles and a body.

## Description

### [Technical Field]

The technology disclosed the present specification relates to a nanoparticle providing medium for hyperthermia and a hyperthermia system using the same, and more particularly, to a nanoparticle providing medium configured to be able to obtain a thermal effect by providing the nanoparticles together when applying an alternating magnetic field to a target region of the human body to treat various diseases of the body and provide, and to block contact between the nanoparticles and the body in order to prevent the nanoparticles from being absorbed into the body, and a hyperthermia system using the same.

### [Background Art]

Methods such as tumor tissue removal surgery, radiotherapy and chemotherapy are widely used in the treatment of malignant or benign tumors, but these treatment methods have the disadvantage of causing side effects that are harmful to the human body at the same time as the treatment of the tumors. Specifically, when the tumor tissue is not clearly demarcated from the normal tissue, it may not be possible to completely remove the tumor tissue by surgery, and in the case of radiotherapy, side effects such as hair loss may occur because it also affects proliferating normal cells. In addition, even in the case of chemotherapy, since complex side effects such as dizziness, vomiting and decreased physical strength may be caused, there is a need for a treatment method capable of minimizing these side effects.

In addition, there is a need for research that expects hyperthermia to have a significant effect as a method of treating Parkinson's disease, Alzheimer's disease, various psychiatric disorders, pain, consciousness disorders, and stress disorder after stroke, in addition to tumor treatment.

Accordingly, when nanoparticles are administered to a treatment target site and an alternating magnetic field is applied to the site, heat is generated from the nanoparticles responding to the alternating magnetic field, and thus, methods of treating various diseases through this heating effect have been conventionally used. However, there were disadvantages that nanoparticles such as iron oxide particles may not be recovered when they are administered into the human body, and thus, there is a possibility that another fatal side effect may be caused, and it is unavoidable that the site is darkly colored due to the characteristics of the nanoparticles even in appearance.

The technology related to the method of treating diseases is a problem to be solved first in modern society based on the expectations of people who want to maintain a healthy life, and the need for the development of technologies to minimize side effects on the human body in the process of treating various diseases is growing. Therefore, there is a continuous demand for the development of a medium that solves the problems of conventional radiotherapy or treatment methods using nanoparticles and effectively provides nanoparticles to the treatment target site while the nanoparticles are not absorbed into the human body and contact is blocked so as to minimize side effects on the human body.

### [Disclosure]

### [Technical Problem]

The technology disclosed in the present specification provides a nanoparticle providing medium that effectively delivers nanoparticles to the treatment target site while isolating them without being absorbed from the human body in order to minimize the side effects due to the absorption of nanoparticles into the human body in hyperthermia of various diseases.

In addition, the technology disclosed in the present specification provides a hyperthermia system using a nanoparticle providing medium, comprising a nanoparticle providing medium with reduced side effects and an alternating magnetic field generating device, wherein the alternating magnetic field generating device applies an alternating magnetic field to the nanoparticles to perform hyperthermia.

The technical problems to be achieved by the nanoparticle providing medium and the hyperthermia system using the same according to the technical idea of the technology disclosed in the present specification are not limited to the above-mentioned problems to be solved, and other problems not mentioned will be clearly understood by those skilled in the art from the following description.

### [Technical Solution]

The nanoparticle providing medium for hyperthermia according to an embodiment of the technology disclosed in the present specification is characterized in that it is formed so as to capture nanoparticles in a region or one or more divided regions and block contact between the nanoparticles and the body.

In addition, the nanoparticle providing medium for hyperthermia according to another embodiment of the technology disclosed in the present specification further comprises a cap coupled to one end of the nanoparticle providing medium.

In addition, the nanoparticles are characterized in that they are made of one or more materials capable of self-heating.

In addition, the nanoparticle providing medium for hyperthermia is characterized in that it is made of one or more of polycaprolactone, poly(ethylene-vinyl acetate), polydioxanone and silicone.

The nanoparticle providing medium for hyperthermia according to another embodiment of the technology disclosed in the present specification further comprises a sensor for detecting temperature, pressure, oxygen saturation, pH, and the like of the surface of the nanoparticle providing medium.

The nanoparticle providing medium for hyperthermia according to an embodiment of the technology disclosed in the present specification further comprises a wireless communication module for performing wireless communication with an external control device.

In addition, the nanoparticle providing medium for hyperthermia may be configured in the shape of a tube or a catheter.

In addition, the nanoparticle providing medium for hyperthermia may be configured in the shape of a plate or a patch.

The hyperthermia system using a nanoparticle providing medium according to an embodiment of the technology disclosed in the present specification is characterized in that it comprises the nanoparticle providing medium and an alternating magnetic field generating device, wherein the alternating magnetic field generating device applies the alternating magnetic field to the nanoparticle providing medium, and the nanoparticle providing medium is configured to block contact between the nanoparticles and a body.

### [Advantageous Effects]

According to the nanoparticle providing medium for hyperthermia and the system using the same according to an embodiment of the technology disclosed in the present specification, by enabling the nanoparticles to be removed again after treatment without completely administering the nanoparticles required for hyperthermia used for the treatment and symptom relief of various diseases into the human body, side effects occurring during the treatment process may be effectively prevented.

According to the nanoparticle providing medium for hyperthermia and the system using the same according to the embodiment of the technology disclosed in the present specification, by further comprising a cap at one end of the nanoparticle providing medium, there is an effect that the contact is blocked so that the nanoparticles are not absorbed into the human body, so the treatment is carried out more safely. In addition, by further comprising a wireless communication module in the cap or its periphery, data measured from the sensor for measuring the temperature, pressure, oxygen saturation, pH, and the like of the surface of the nanoparticle providing medium may be transmitted to an external control device, and based on these data, the external control device may efficiently control the strength, frequency and duration of the applied alternating magnetic field.

In addition, by selecting the material for the nanoparticle providing medium from various materials harmless to the human body, and comprising the composition of the nanoparticles with several types of materials capable of self-heating such as, but not limited to, manganese/zinc alloy, iron oxide, magnesium, cobalt, nickel, calcium and sodium, and combining them in an appropriate ratio, a more excellent heating effect may be obtained.

In addition, according to another embodiment of the technology disclosed in the present specification, by allowing the nanoparticle providing medium to be configured in the shape of a tube or a catheter, or in the shape of a plate or a patch so that it may be applied to various sites of the human body and to be grafted to any site requiring hyperthermia, there is the effect that the versatility of the medium is increased.

On the other hand, the effects that may be achieved by the nanoparticle providing medium for hyperthermia and the hyperthermia system through the nanoparticle providing medium according to an embodiment of the technology disclosed in the present specification are not limited to those described above, and other effects not mentioned will be clearly understood by those skilled in the art from the following description.

### [Description of Drawings]

In order to more fully understand the drawings cited in present specification, a brief description of each drawing is provided.
Figure 1 shows a nanoparticle providing medium for hyperthermia configured in the shape of a tube or a catheter and nanoparticles captured in one or more divided regions therein as an embodiment of the technology disclosed in the present specification.
Figure 2 shows that a cap is further comprised at one end of a nanoparticle providing medium configured in the shape of a tube or a catheter.
Figure 3 shows a structure comprising a cap, a wireless communication module and a sensor in a nanoparticle providing medium configured in the shape of a tube or a catheter.
Figure 4 shows that a nanoparticle providing medium is made in the shape of a plate or a patch as another embodiment of the technology disclosed in the present specification, and nanoparticles captured in one or more divided regions in the nanoparticle providing medium.
Figure 5 shows that a cap is further comprised in a nanoparticle providing medium configured in the shape of a plate or a patch.
Figure 6 shows an embodiment further comprising a cap, a wireless communication module and a sensor in a nanoparticle providing medium configured in the shape of a plate or a patch.
Figure 7 shows the configuration of a hyperthermia system in which a nanoparticle providing medium in the shape of a catheter or a tube is inserted into the human body and an alternating magnetic field generating device applies an alternating magnetic field toward the inserted site to perform hyperthermia.
Figure 8 shows the configuration of a hyperthermia system in which a nanoparticle providing medium in the shape of a plate or a patch is inserted into the human body and an alternating magnetic field generating device applies an alternating magnetic field toward the inserted site to perform hyperthermia.

### [Mode for Carrying out the Invention]

Since the technology disclosed in the present specification may have various modifications and may have various embodiments, specific embodiments will be illustrated in the drawings and described in detail though the detailed description. However, this is not intended to limit the technology disclosed in the present specification to specific embodiments, it is to be understood that the technology disclosed in the present specification includes all modifications, equivalents, and substitutes included in the spirit and technical scope of the technology disclosed in the present specification.

In describing the technology disclosed in the present specification, if it is determined that the specific description of the related known technologies may unnecessarily obscure the gist of the technology disclosed in the present specification, the detailed description thereof will be omitted. In addition, numbers (for example, 1st, 2nd, and the like) used in the description process of the present specification are only identification symbols for distinguishing one component from other components.

In addition, when it mentioned that a component is "connected" or "coupled" to another component in the present specification, it is to be understood that the component may be directly connected or coupled to the other component, but may be connected or combined through another component in the middle unless otherwise specifically stated.

In addition, in the present specification, a component expressed as "... part" may mean that two or more components may be combined into one component, or one component may be divided into two or more for each more subdivided function. In addition, it goes without saying that each of the components to be described below may additionally perform some or all of the functions of other components in addition to the main functions that each component is responsible for, and some of the main functions that each component is responsible for may be exclusively performed by other component.

Expressions such as "1st", "2nd", "first", or "second" used in various embodiments may modify various components regardless of order and/or importance, and do not limit the components. For example, without departing from the scope of the technology disclosed in the present specification, a first component may be referred to as a second component, and similarly, a second component may also be renamed to a first component.

Hereinafter, a nanoparticle providing medium for hyperthermia according to a preferred embodiment and a system using the same will be described in detail.

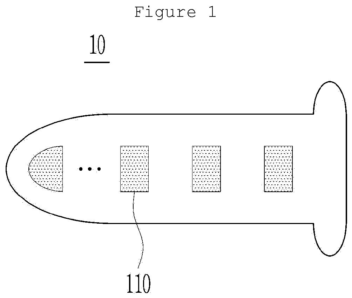

Referring to Figure 1, the nanoparticle providing medium 10 for hyperthermia according to an embodiment of the present invention may be configured in the shape of a tube or a catheter, and the nanoparticles may be respectively captured at positions divided into a plurality of partitioned regions 110 in the nanoparticle providing medium 10. This nanoparticle providing medium 10 may be made of a material harmless to the human body, such as polycaprolactone (PCL), poly(ethylene-vinyl acetate) (PEVA), polydioxanone (PDO) and silicone (SE-1700), and may be composed of other different materials. In addition, its size or length may be appropriately adjusted to suit the site to be treated.

The nanoparticle providing medium 10 may be used for hyperthermia, and prevents various unpredictable side effects caused by the nanoparticles by completely blocking contact with each other so that the nanoparticles are not absorbed into the human body. Specifically, since the nanoparticles have a dark color, when injected into the human body as it is, the corresponding site cannot be prevented from being colored black, and these nanoparticles are absorbed into the human body and may cause inflammation in the process of movement or may cause other serious side effects. Therefore, the nanoparticle providing medium 10 is configured to block any contact so that the nanoparticles are not absorbed into the human body, thereby providing the effect of preventing the side effects described above in advance.

In addition, by designating one or more partitioned regions 110 inside the nanoparticle providing medium 10, the nanoparticles may be captured in each divided region 110 of the nanoparticle providing medium 10. Accordingly, the hyperthermia effect may be greatly increased by providing the heating effect through the nanoparticles to a wide site without concentrating on one region.

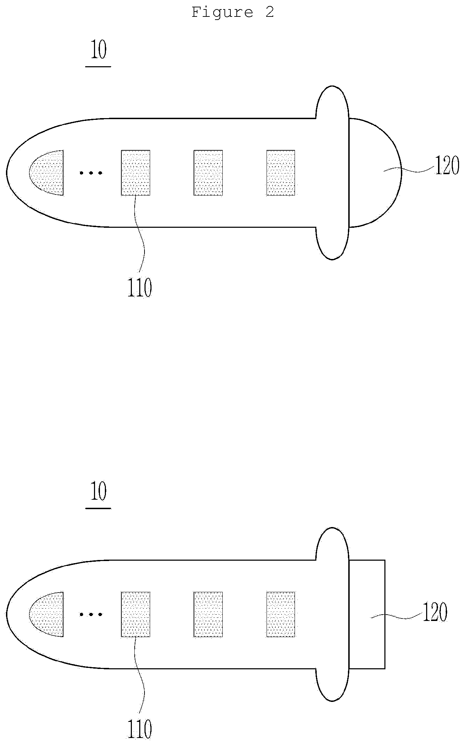

Figure 2 shows an embodiment in which a cap 120 is further included in the nanoparticle providing medium 10 configured in the shape of a tube or a catheter.

According to Figure 2, the cap 120 is coupled to one end of the nanoparticle providing medium 10, and serves to prevent the nanoparticles from leaking or flowing to the outside. The cap 120 of the nanoparticle providing medium 10 may be configured in a dome shape or an angled shape as shown in Figure 2 but may be configured in any shape other than these, and the shape of the cap 120 is not limited to the embodiment as shown in Figure 2.

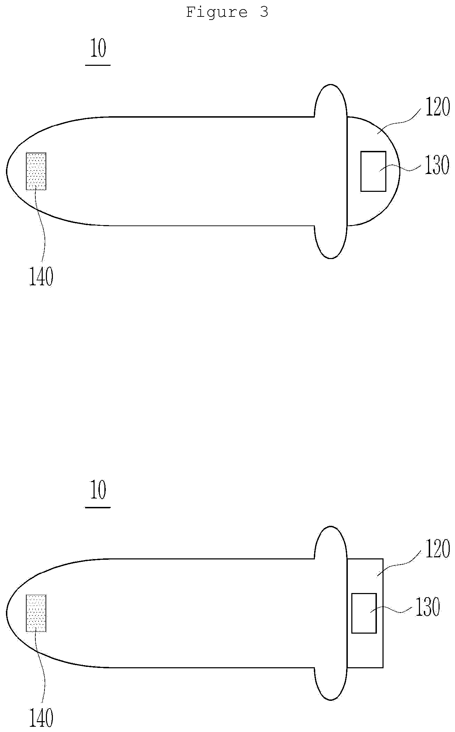

Figure 3 shows an embodiment in which the cap 120, the wireless communication module 130 and the sensor 140 are further provided in the nanoparticle providing medium 10 configured in the shape of a tube or a catheter.

The wireless communication module 130 may be further included in a location or a periphery of the cap 120. The wireless communication module 130 may receive data such as temperature, pressure, oxygen saturation, pH, and the like of the surface of the nanoparticle providing medium 10 measured from the sensor 140 included in the nanoparticle providing medium 10, and transmit the data towards an external control device. Such data may be appropriately utilized in the course of hyperthermia and used to control treatment duration, strength and frequency of the magnetic field.

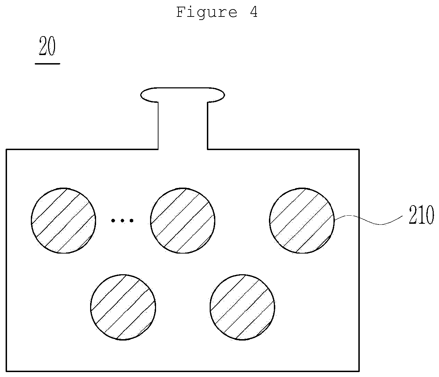

Figure 4 shows a nanoparticle providing medium 20 configured in the shape of a plate or a patch.

According to Figure 4, the nanoparticle providing medium 20 is formed to completely block the contact between the nanoparticles and the human body, as shown in Figure 1, and the material may be made of polycaprolactone (PCL), poly(ethylene-vinyl acetate) (PEVA), polydioxanone (PDO) and silicone (SE-1700), which are harmless to the human body as in the nanoparticle providing medium 10 of Figure 1, and may be composed of other different materials. In addition, as in the nanoparticle providing medium 10 of Figure 1, its size or length may be appropriately adjusted to suit the site to be treated.

Likewise, one or more partitioned regions 210 are provided inside the nanoparticle providing medium 20, and nanoparticles are captured in these regions 210, respectively. Thus, when an alternating magnetic field is applied to widely spaced nanoparticles, a uniform thermal effect may maintained on the site to be treated, so that the hyperthermia effect may be remarkably improved.

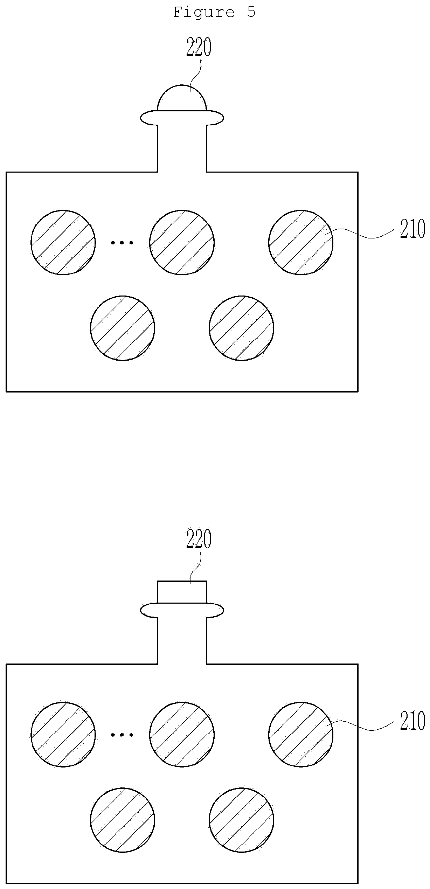

Figure 5 shows an embodiment in which a cap 220 is further included in the nanoparticle providing medium 20 configured in the shape of a plate or a patch.

According to Figure 5, the cap 220 is coupled to one end of the nanoparticle providing medium 20, and serves to prevent the nanoparticles from leaking or flowing to the outside. The cap 220 of the nanoparticle providing medium 20 may be configured in a dome shape or an angled shape as shown in Figure 5 but may be configured in any shape other than these, and the shape of the cap 220 is not limited to the embodiment as shown in Figure 2.

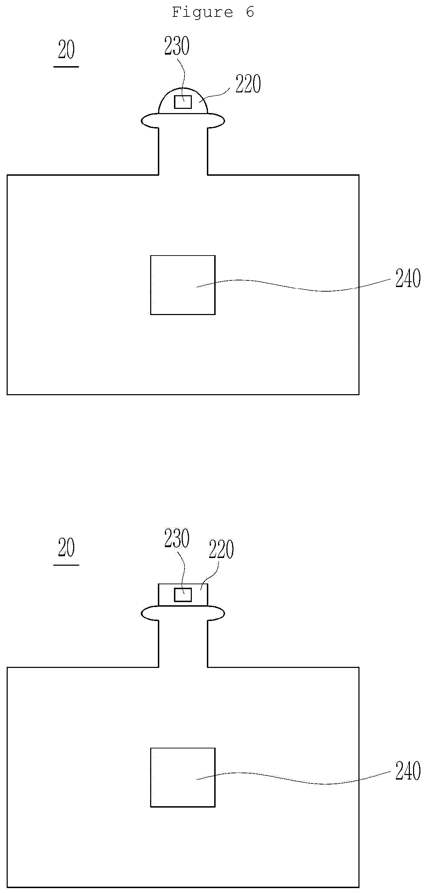

Figure 6 shows an embodiment in which the cap 220, the wireless communication module 230 and the sensor 240 are further provided in the nanoparticle providing medium 20 configured in the shape of a plate or a patch.

The wireless communication module 230 may be further included in a location or a periphery of the cap 220. The wireless communication module 230 performs an operation of receiving data such as temperature, pressure, oxygen saturation, pH, and the like of the surface of the nanoparticle providing medium 20 measured from the sensor 240 included in the nanoparticle providing medium 20, and transmitting the data towards an external control device, as described in Figure 3. Likewise, such data is appropriately utilized in the course of hyperthermia and used to control treatment duration, strength and frequency of the magnetic field.

Hereinafter, a hyperthermia system using a nanoparticle providing medium according to another embodiment including the above configuration will be described.

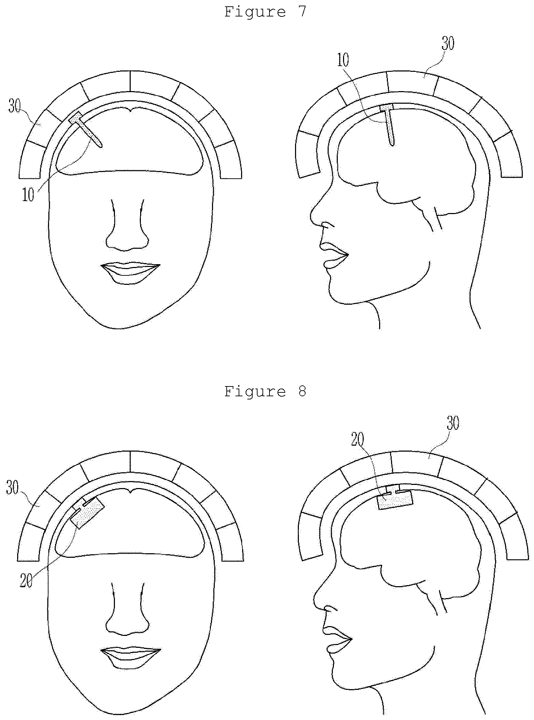

Figure 7 shows the configuration of a hyperthermia system using a nanoparticle providing medium, further comprising an alternating magnetic field generating device 30 for applying an alternating magnetic field to the nanoparticle providing medium 10 configured in the shape of a catheter or a tube, wherein thermal treatment is performed through an alternating magnetic field applied from such alternating magnetic field generating device 30.

According to Figure 7, the nanoparticle providing medium 10 injected by dividing the nanoparticles into the partitioned regions is inserted into the target site to be treated, and the alternating magnetic field generating device 30 appropriately applies an alternating magnetic field to such nanoparticle providing medium 10. When an alternating magnetic field is applied to the magnetic nano-medium, an excellent temperature rise phenomenon is observed for nanoparticles as described in detail in (Patent Document 1) KR 10-2017-0115951 A, referenced in the present specification. Therefore, it is possible to treat various diseases such as tumors through the thermal effect of these nanoparticles, and in addition, the nanoparticle providing medium 10 according to an embodiment disclosed in the present specification may play a role of completely blocking various side effects triggered by nanoparticles that may occur during such a treatment process.

In the hyperthermia system using the nanoparticle providing medium, the alternating magnetic field generating device 30 applies a low-frequency alternating magnetic field harmless to the human body, and the strength and frequency of the alternating magnetic field may be appropriately controlled to obtain a target temperature. When an alternating magnetic field is applied, the nanoparticles self-heat by spin rotation, and the symptoms of various diseases may be improved by hyperthermia stimulation of cancer tissue or deep brain that is more sensitive to heat.

Specifically, normal cells are killed at a temperature of about 46°C. or higher, but cancer cells may be effectively killed even at a temperature of about 42°C or higher because they have heat-sensitive properties. Therefore, the hyperthermia system through the nanoparticle providing medium disclosed in the present specification may properly treat a tumor such as cancer by generating an appropriate thermal effect in a site requiring treatment, and has a therapeutic effect with minimal side effects.

In addition, when deep brain hyperthermia stimulation is used as shown in Figure 7, in addition to the treatment of the tumors described above, a useful effect may also be provided for indications such as abnormal movements such as Parkinson's disease, essential tremor (hand tremor) and dystonia, mental disorders such as dementia or Alzheimer's disease, depression, obsessive compulsive disorder, Tourette syndrome and schizophrenia, various pains of unknown cause, disturbance of consciousness, and stress disorder after stroke (cerebral infarction, cerebral hemorrhage), similar to the effect of the existing deep brain stimulator. The existing deep brain stimulator is a method of treating abnormal sites by stimulating abnormally activated cells with high-frequency electrodes, so that there were disadvantages in that damage to surrounding cells may be caused and the risk of using high-frequency electrodes increases. Therefore, it will be said that the hyperthermia system using the nanoparticle providing medium 10 as described above provides a safer and more useful effect in the treatment of various indications described above.

Figure 8 shows the configuration of a hyperthermia system using a nanoparticle providing medium, further comprising an alternating magnetic field generating device 30 for applying an alternating magnetic field to the nanoparticle providing medium 20 configured in the shape of a plate or a patch, wherein thermal treatment is performed through an alternating magnetic field applied from such alternating magnetic field generating device 30.

According to Figure 8, as in the configuration of the system of Figure 7, the nanoparticle providing medium 20 injected by dividing the nanoparticles into the partitioned regions is inserted into the target site to be treated, and the alternating magnetic field generating device 30 appropriately applies an alternating magnetic field to such nanoparticle providing medium 20. When an alternating magnetic field is applied to the magnetic nano-medium, an excellent temperature rise phenomenon is observed for nanoparticles. Therefore, it is possible to improve the symptoms of various diseases such as tumors through the thermal effect of these nanoparticles, and in addition, the nanoparticle providing medium 20 according to an embodiment disclosed in the present specification may play a role of completely blocking various side effects triggered by nanoparticles that may occur during such a treatment process.

As in the system of Figure 7, in the hyperthermia system using the nanoparticle providing medium as shown in Figure 8, the alternating magnetic field generating device 30 applies a low-frequency alternating magnetic field harmless to the human body, and the strength and frequency of the alternating magnetic field may be appropriately controlled to obtain a target temperature.

In addition, the hyperthermia system using the nanoparticle providing medium as shown in Figure 8 may perform brain cortex hyperthermia stimulation, and has the same indications as in the deep brain stimulation, but may provide a treatment system with further improved versatility that may be applied to various diseases by appropriately inserting it into a site requiring stimulation according to various diseases and expanding the thermal stimulation site more widely.

In addition, although not shown through the drawings of the present specification, the configuration of the hyperthermia system may also be used in treatment methods such as spinal cord hyperthermia stimulation, cauda equina hyperthermia stimulation, and peripheral nerve hyperthermia stimulation, and this treatment methods may be used for indications such as cancerous pain in the spinal cord and deep peripheral nerves, pain after spinal surgery, spasticity after spinal surgery, spasticity due to cerebral palsy, and stress disorder after spinal cord injury. The hyperthermia system effective for such various indications is designed to provide a safer, less side-effect and more efficient treatment method compared to the existing technology.

Although the present invention has been described in detail above, the scope of a right for the present invention is not limited thereto, and it will be apparent to those of ordinary skill in the art that various modifications and changes may be made without departing from the technical spirit of the present invention described in the claims.

## Claims

1. A nanoparticle providing medium for hyperthermia, comprising nanoparticles and one or more divided regions, wherein the nanoparticles are captured in the one or more divided regions and blocked from contact with the human body.

2. The nanoparticle providing medium for hyperthermia according to claim 1, further comprising a cap coupled to one end of the nanoparticle providing medium.

3. The nanoparticle providing medium for hyperthermia according to claim 1, wherein the nanoparticles are made of one or more materials capable of self-heating.

4. The nanoparticle providing medium for hyperthermia according to claim 1, wherein the nanoparticle providing medium for hyperthermia is made of one or more of polycaprolactone, poly(ethylene-vinyl acetate), polydioxanone and silicone, and the size or length of the nanoparticle providing medium may be adjusted according to be treated.

5. The nanoparticle providing medium for hyperthermia according to claim 1, further comprising a wireless communication module configured to perform wireless communication with an external control device.

6. The nanoparticle providing medium for hyperthermia according to claim 1, further comprising a sensor configured to sense temperature, pressure, oxygen saturation or pH of the surface of the nanoparticle providing medium.

7. The nanoparticle providing medium for hyperthermia according to claim 1, wherein the nanoparticle providing medium is configured in the shape of a tube or a catheter.

8. The nanoparticle providing medium for hyperthermia according to claim 1, wherein the nanoparticle providing medium is configured in the shape of a plate or a patch.

9. A hyperthermia system using a nanoparticle providing medium, comprising the nanoparticle providing medium according to any one of claims 1 to 8 and an alternating magnetic field generating device, wherein the alternating magnetic field generating device applies an alternating magnetic field to the nanoparticle providing medium, and the nanoparticle providing medium is configured to block contact between the nanoparticles and the human body.
